# EUROPÄISCHE PATENTANMELDUNG

(11) **EP 0 590 419 A1**
(43) Veröffentlichungstag der Anmeldung: **06.04.1994**
(21) Anmeldenummer: 93114889.4
(22) Anmeldetag: 16.09.1993
(51) Int. Cl.: C07C 209/00, C07C 209/70, C07C 211/11, C07C 211/22, C07D 295/13

(54) **Verfahren zur Herstellung von ungesättigten Aminen**

(30) Priorität: 28.09.1992 DE 4232424
(71) Anmelder: BASF Aktiengesellschaft, D-67063 Ludwigshafen (DE)
(72) Erfinder: Witzel, Tom, Dr., D-6700 Ludwigshafen (DE); Merger, Franz, Dr., D-6710 Frankenthal (DE); Fuchs, Eberhard, Dr., D-6700 Ludwigshafen (DE)

(57) **Zusammenfassung**

Verfahren zur Herstellung von ungesättigten Aminen der allgemeinen Formel I 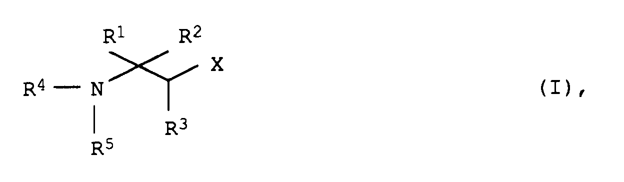
in der
- R¹,R²,R³,R⁴,R⁵: Wasserstoff, C₁- bis C₂₀-Alkyl, C₂- bis C₂₀-Alkenyl, C₃- bis C₈-Cycloalkyl, C₃- bis C₈-Cycloalkenyl, Phenyl, C₇- bis C₂₀-Phenylalkyl, C₁- bis C₂₀- Alkoxy, Phenoxy, C₇- bis C₂₀-Phenoxyalkyl, C₁- bis C₂₀-Alkylamino, Phenylamino, C₇- bis C₂₀-Phenylalkylamino, Pyridyl, Imidazolyl oder R¹ und R² oder R¹ und R³ oder R² und R³ gemeinsam für ein gegebenenfalls ein- bis fünffach durch C₁- bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen und
- X: =C-N(R⁴R⁵) oder -C=N-R⁴,
durch Umsetzung von 2-Alkenalen der allgemeinen Formel II 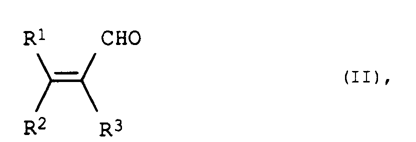
in der R¹, R² und R³ die oben genannten Bedeutungen haben, mit primären und sekundären Aminen der allgemeinen Formel III 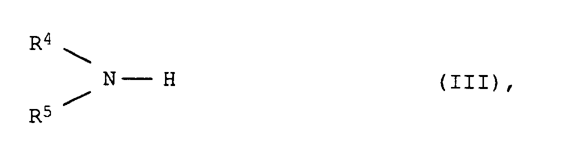
in der R⁴ und R⁵ die oben genannten Bedeutungen haben, bei Temperaturen von (-20) bis 70°C und Drücken von 0,01 bis 100 bar, indem man die Umsetzung ohne Abtrennung des Reaktionswassers durchführt sowie deren Umaminierung und Hydrierungen der ungesättigten Amine neue 1,3-Diamine der allgemeinen Formel IV 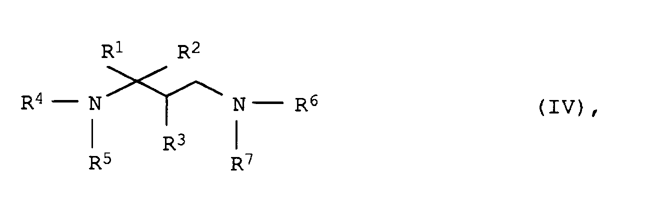
in der
R¹,R²,R³,R⁴,R⁵,R⁶,R⁷
Wasserstoff, C₁- bis C₂₀-Alkyl, C₂- bis C₂₀-Alkenyl, C₃- bis C₈-Cycloalkyl, C₃- bis C₈-Cycloalkenyl, Phenyl, C₇- bis C₂₀-Phenylalkyl, C₁- bis C₂₀- Alkoxy, Phenoxy, C₇- bis C₂₀-Phenoxyalkyl, C₁- bis C₂₀-Alkylamino, Phenylamino, C₇- bis C₂₀-Phenylalkylamino, Pyridyl, Imidazolyl oder R¹ und R² oder R¹ und R³ oder R² und R³ gemeinsam für ein gegebenenfalls ein- bis fünffach durch C₁- bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen
bedeuten, mit der Maßgabe, daß R¹ und R² nicht für Wasserstoff stehen, wenn R³ Methyl und R⁴ und R⁵, R⁶ und R⁷ jeweils Wasserstoff oder Methyl, R⁴ und R⁶ Wasserstoff und R⁵ und R⁷ Ethyl sowie R⁴ und R⁵, R⁶ und R⁷ jeweils zusammen (CH₂)₅ oder (CH₂)₂-O-(CH₂)₂ bedeuten.

## Beschreibung

Die vorliegende Erfindung betrifft ein neues Verfahren zur Herstellung von 1,3-Diaminopropenen durch Umsetzung von 2-Alkenalen mit primären und sekundären Aminen ohne Abtrennung des Reaktionswassers.

Aus "Acrolein", Wiley & Sons, N.Y., Chapter 6, Seiten 96 bis 109 (1975), sind Reaktionen primärer und sekundärer aliphatischer Amine mit Acrolein zu alkylsubstituierten 1-Propen-1,3-diaminen bekannt. Nach diesem Verfahren bilden sich aus zwei mol Amin mit einem mol Acrolein unter Wasserabspaltung in Gegenwart eines wasserentziehenden Mittels oder eines nicht mit Wasser mischbaren Lösungsmittels N,N'-Dialkyl-1-propen-1,3-diamine.

Aus der US-A-2 565 488 sind N,N'-Dialkyl-1-propen-1,3-diamine und deren Herstellung aus 2-Alkenalen und primären Aminen unter Wasserausschluß-Bedingungen in Gegenwart von wasserentziehenden Mitteln wie Kaliumcarbonat bekannt.

Die Addition von Di-n-propylamin an Acrolein ist aus der EP-A-424 171 bekannt. Auch bei diesem Verfahren muß das Reaktionswasser entweder durch Verwendung eines Trockenmittels oder alternativ durch Verwendung eines mit Wasser nicht mischbaren Lösungsmittels und Abtrennen der wäßrigen Phase entfernt werden.

Die bisher bekannten Verfahren zur Herstellung von alkyl-substituierten 1-Propen-1,3-diaminen aus 2-Alkenalen mit primären und sekundären aliphatischen Aminen haben den Nachteil, daß unter Zusatz eines wasserentziehenden Mittels oder eines mit Wasser nicht mischbaren Lösungsmittels und Abtrennung einer wäßrigen Phase gearbeitet werden muß und nur mäßige Ausbeuten erreicht werden.

Der vorliegenden Erfindung lag daher die Aufgabe zugrunde, den zuvor genannten Nachteilen abzuhelfen.

Demgemäß wurde ein neues und verbessertes Verfahren zur Herstellung von ungesättigten Aminen der allgemeinen Formel I 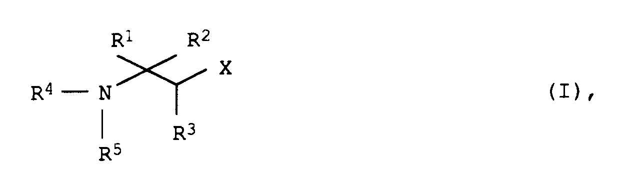
in der
- R¹,R²,R³,R⁴,R⁵: Wasserstoff, C₁- bis C₂₀-Alkyl, C₂- bis C₂₀-Alkenyl, C₃- bis C₈-Cycloalkyl, C₃- bis C₈-Cycloalkenyl, Phenyl, C₇- bis C₂₀-Phenylalkyl, C₁- bis C₂₀- Alkoxy, Phenoxy, C₇- bis C₂₀-Phenoxyalkyl, C₁- bis C₂₀-Alkylamino, Phenylamino, C₇- bis C₂₀-Phenylalkylamino, Pyridyl, Imidazolyl oder R¹ und R² oder R¹ und R³ oder R² und R³ gemeinsam für ein gegebenenfalls ein- bis fünffach durch C₁-bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen und
- X: =C-N(R⁴R⁵) oder -C=N-R⁴,
durch Umsetzung von 2-Alkenalen der allgemeinen Formel II 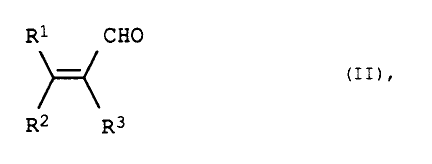
in der R¹, R² und R³ die oben genannten Bedeutungen haben, mit Ammoniak, primären und sekundären Aminen der allgemeinen Formel III 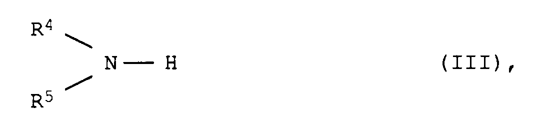
in der R⁴ und R⁵ die oben genannten Bedeutungen haben, bei Temperaturen von (-20) bis 70°C und Drücken von 0,01 bis 100 bar gefunden, welches dadurch gekennzeichnet ist, daß man die Umsetzung ohne Abtrennung des Reaktionswassers durchführt sowie deren Umaminierung zu den Verbindungen I' und deren Hydrierung zu 1,3-Diaminen und neue 1,3-Diamine der allgemeinen Formel IV 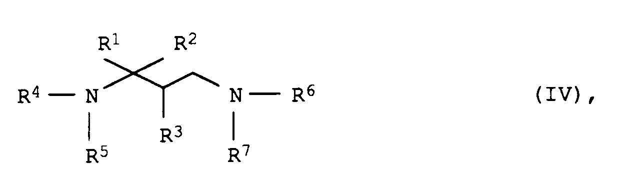
in der
R¹,R²,R³,R⁴,R⁵,R⁶,R⁷
Wasserstoff, C₁- bis C₂₀-Alkyl, C₂- bis C₂₀-Alkenyl, C₃- bis C₈-Cycloalkyl, C₃- bis C₈-Cycloalkenyl, Phenyl, C₇- bis C₂₀-Phenylalkyl, C₁- bis C₂₀- Alkoxy, Phenoxy, C₇- bis C₂₀-Phenoxyalkyl, C₁- bis C₂₀-Alkylamino, Phenylamino, C₇-bis C₂₀-Phenylalkylamino, Pyridyl, Imidazolyl oder R¹ und R² oder R¹ und R³ oder R² und R³ gemeinsam für ein gegebenenfalls ein- bis fünffach durch C₁- bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen
bedeuten, mit der Maßgabe, daß R¹ und R² nicht für Wasserstoff stehen, wenn R³ Methyl und R⁴ und R⁵, R⁶ und R⁷ jeweils Wasserstoff oder Methyl, R⁴ und R⁶ Wasserstoff und R⁵ und R⁷ Ethyl sowie R⁴ und R⁵, R⁶ und R⁷ jeweils zusammen (CH₂)₅ oder (CH₂)₂-O-(CH₂)₂ bedeuten.

Das erfindunsgemäße Verfahren läßt sich wie folgt durchführen:

### - Herstellung von ungesättigten Aminen I

Man kann das 2-Alkenal II mit Ammoniak, einem primären oder sekundären Amin III entweder diskontinuierlich oder kontinuierlich bei Temperaturen von (-20) bis 70°C, bevorzugt 20 bis 50°C und Drücken von 0,1 bis 10 bar, bevorzugt 1 bis 5 bar, besonders bevorzugt Normaldruck (Atmosphärendruck) umsetzen. Lediglich bei gasförmigen Aminen III kann es notwendig sein, Drücke von 2 bis 300 bar, bevorzugt 5 bis 200 bar zu verwenden. Im Falle des Einsatzes eines primären Amins III oder III' erhält man ungesättigte Amine I oder I', in denen X = C=N-R⁴ oder X' = C=N-R⁶ bedeutet. Beim Einsatz eines sekundären Amins III oder III' erhält man ungesättigte Amine I oder I' in denen X =C-N(R⁴R⁵) oder X' = =C-N-(R⁶,R⁷) bedeutet.

Das Molverhältnis vom primären oder sekundären Amin III zum 2-Alkenal II sollte mindestens 2 : 1 betragen, in der Regel zwischen 2 : 1 und 50 : 1, bevorzugt 2 : 1 bis 10 : 1, besonders bevorzugt 3 : 1 bis 5 : 1.

2-Alkenale der allgemeinen Formel II sind beispielsweise:
Acrolein, Methacrolein, Crotonaldehyd, 2-Methylcrotonaldehyd, 3-Methylcrotonaldehyd, 2-Ethylacrolein, 2-Propylacrolein, 2-iso-Propylacrolein, 2-Nonylacrolein, 2-Ethylhexenal, 2-Propylheptenal, 1-Cyclopentencarbaldehyd, 1-Cyclohexencarbaldehyd und Zimtaldehyd.

### - Umaminierung der ungesättigten Aminen I

Die aus Ammoniak, primären bzw. sekundären Aminen III mit 2-Alkenalen II erhaltenen ungesättigten Aminen I können mit primären bzw. sekundären Aminen III' zu ungesättigten Aminen I' direkt und ohne Entfernen des Reaktionswassers oder überschüssigen Amins III eingesetzt werden. Es kann aber auch das ungesättigte Amine I als Reinprodukt verwendet werden.

Der Austausch kann durch Erwärmen der Reaktionslösung auf 20 bis 150°C, bevorzugt 30 bis 120°C, besonders bevorzugt 45 bis 90°C beschleunigt und vervollständigt werden. Dabei wird der Stickstoff, der der Doppelbindung am nächsten steht, bevorzugt ausgetauscht. Besonders bevorzugt ist der Austausch gegen ein primäres bzw. sekundäres Amin III', dessen Siedepunkt höher liegt, als der des abzuspaltenden Amins der allgemeinen Formel I. Bevorzugt arbeitet man bei einem Druck, bei dem der Siedepunkt des abzuspaltenen Amins bei 50 bis 80°C liegt, also bei Drücken von 0,01 bis 3 bar, bevorzugt 0,05 bis 1,5 bar besonders bevorzugt 0,1 bis 0,7 bar.

Zur Herstellung von ungesättigten Aminen I und zur Herstellung von ungesättigten Aminen I' durch Umaminierung können Ammoniak, primäre oder sekundäre Amine III und III' eingesetzt werden. Im folgenden seien die wichtigsten zusammengestellt:

### Ammoniak.

Primäre Amine III und III' sind beispielsweise:
Primäre Amine ohne weitere Heteroatome sind beispielsweise: Methylamin, Ethylamin, n-Propylamin, iso-Propylamin, n-Butylamin, 2-Methyl-propylamin, tert.-Butylamin, n-Pentylamin, 2-Methyl-butylamin, 3-Methyl-butylamin, 2,2-Dimetyl-propylamin, 3,3-Dimethyl-propylamin, n-Hexylamin, n-Octylamin, 2-Ethyl-hexylamin, Tridecylamin, Allylamin, Crotylamin, Methallylamin, Cyclopropylamin, Cyclopentylamin, Cyclohexylamin, Benzylamin und Anilin.

Mehrfach funktionalisierte Amine III und III' mit mindestens einem primären Aminorest sind beispielsweise:
Ethylendiamin, 1,3-Propylendiamin, 1,4-Butylendiamin, 1,5-Pentamethylendiamin, 1,6-Hexamethylendiamin, Isophorondiamin, 2-Butyl-2-ethyl-1,5-pentandiamin, 1,2-Propylendiamin, 2,2-Dimethyl-1,3-propylendiamin, 4,4'-Diaminodicyclohexylmethan, 3,3'-Dimethyl-4,4'-diaminodicyclohexylmethan, 4,4'-Diaminodiphenylmethan, N-Methylethylendiamin, N,N-Dimethylethylendiamin, N,N-Diethylethylendiamin, N-Methyl-1,3-propylendiamin, N,N-Dimethyl-1,3-propylendiamin, N,N-Diethyl-1,3-propylendiamin, N-Cyclohexyl-1,3-propylendiamin, 1-Diethylamino-4-aminopentan, Diethylentriamin, N,N-Dimethylethylentriamin, N,N-Dimethylpropylentriamin, N-(2-Aminoethyl)-1,3-propandiamin, N,N'-Bis(3-aminopropyl)-ethylendiamin und N,N-Dimethyl-1,6-hexamethylendiamin.

Primäre Amine III und III' mit weiteren Heteroatomsubstituenten sind beispielsweise:
2-Aminoethanol, 1-Amino-2-propanol, 2-(2-Aminoethoxy)-ethanol, 3-Aminopropanol, 2-Amino-1-butanol, 4,9-Dioxadodecan-1,12-diamin, 4,7,10-Trioxatridecan-1,13-diamin, 2-Methoxyethylamin, 2-Ethoxyethylamin, 3-Methoxypropylamin, 3-Ethoxypropylamin, 3-(2-Ethylhexoxy)propylamin, 3-(2-Methoxyethoxy)propylamin, 2-Methoxyphenylethylamin, 4-Methoxyphenylethylamin und Homoveratrylamin.

Sekundäre Amine III und III' sind beispielsweise:
Sekundäre Amine ohne weitere Heteroatome sind beispielsweise: Dimethylamin, Diethylamin, Di-n-propylamin, Di-iso-propylamin, Di-n-butylamin, Di-(2-methyl-propyl)-amin, Di-tert.-butylamin, Di-n-pentylamin, Di-(2-methyl-butyl)-amin, Di-(3-methyl-butyl)-amin, Di-(2,2-dimetylpropyl)-amin, Di-(3,3-dimethyl-propyl)-amin, Di-n-hexylamin, Di-2-ethyl-hexylamin, Di-tridecylamin, N-Methylbutylamin, N-Ethylbutylamin, Diallylamin, Dicrotylamin, Dimethallylamin, Dicyclopropylamin, Dicyclopentylamin, N-Methyl-cyclohexylamin, N-Ethyl-cyclohexylamin, Dicyclohexylamin, N-Methyl-anilin, N-Ethylanilin und Diphenylamin.

Sekundäre Amine III und III' mit weiteren Heteroatomsubstituenten sind beispielsweise:
Diethanolamin, Di-isopropanolamin, Methylethanolamin, Ethylethanol- amin, Butylethanolamin, Cyclohexylethanolamin, Hydroxyethylanilin und Di-2-methoxyethylamin.

### - Hydrierung von ungesättigten Aminen

Die aus der Umsetzung von Ammoniak, primären oder sekundären Aminen III und 2-Alkenalen II erhaltenen ungesättigten Amine I können ohne Aufarbeitung und in Gegenwart des Reaktionswassers an gängigen Hydrierkatalysatoren wie Palladium, Platin, Platin, Ruthenium, Rhodium, Kobalt, Nickel und Kupfer als Metall oder Metallgemisch oder auf Trägermaterial wie Aluminiumoxid, Siliciumoxid, Aktivkohle, Zirkonoxid oder Titandioxid, insbesondere an Palladium als Metall oder auf Trägermaterial wie Aluminiumoxid, Siliciumoxid oder Aktivkohle, bevorzugt ohne Aufarbeitung und ohne Entfernen des Reaktionswassers zu den entsprechenden gesättigten, 1,3-Diaminen IV hydriert werden. Gleichermaßen lassen sich die durch Aminaustauschreaktion hergestellten ungesättigten Amine I', bevorzugt ohne Aufarbeitung des Rohprodukts hydrieren.

Die Hydrierung kann gegebenenfalls in inerten Lösungsmitteln wie Ethern, beispielsweise Methyl-tert.-butylether, Tetrahydrofuran und Dioxan oder Estern, beispielsweise Essigsäuremethylester und Essigsäureethylester bei Temperaturen von 0 und 150°C, bevorzugt 10 und 80°C, besonders bevorzugt 30 und 60°C durchgeführt werden. Der Wasserstoffdruck beträgt zwischen 1 und 350 bar, bevorzugt zwischen 1 und 100 bar.

Die Substituenten R¹, R², R³, R⁴, R⁵, R⁶ und R⁷ in den Verbindungen der allgemeinen Formeln I, I', II, III, III' und IV haben folgende Bedeutungen:
R¹,R²,R³,R⁴,R⁵,R⁶,R⁷

### - Wasserstoff,

- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₂- bis C₂₀-Alkenyl, bevorzugt C₂- bis C₁₂-Alkenyl wie Ethenyl, n-Propenyl, iso-Propenyl, n-Butenyl, iso-Butenyl, sec.-Butenyl, tert.-Butenyl, n-pentenyl, iso-pentenyl, sec.-Pentenyl, neo-pentenyl, 1,2-Dimethenyl-propenyl, n-Hexenyl, iso-Hexenyl, sec.-Hexenyl, n-Heptenyl, iso-Heptenyl, n-Octenyl, iso-octenyl, n-Nonenyl, iso-Nonenyl, n-Decenyl, iso-Decenyl, n-Undecenyl, iso-Undecenyl, n-Dodecenyl und iso-Dodecenyl, besonders bevorzugt C₂- bis C₄-Alkenyl wie Ethenyl, n-Propenyl, iso-Propenyl, n-Butenyl, iso-Butenyl, sec.-Butenyl und tert.-Butenyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- C₃- bis C₈-Cycloalkenyl wie Cyclopropenyl, Cyclobutenyl, Cyclopentenyl, Cyclohexenyl, Cycloheptenyl und Cyclooctenyl, bevorzugt Cyclopentenyl, Cyclohexenyl und Cyclooctenyl,
- Phenyl,
- C₇- bis C₂₀-Phenylalkyl, bevorzugt C₇- bis C₁₂-Phenylalkyl wie Benzyl, 1-Phenethyl, 2-Phenethyl, 1-Phenylpropyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₁- bis C₂₀- Alkoxy, bevorzugt C₁- bis C₁₂-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, n-Nonoxy, iso-Nonoxy, n-Decoxy, iso-Decoxy, n-Undecoxy, iso-Undecoxy, n-Dodecoxy und iso-Dodecoxy, besonders bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy,
- Phenoxy,
- C₇- bis C₂₀-Phenoxyalkyl, bevorzugt C₇- bis C₁₂-Phenoxyalkyl wie Benzoxy, 1-Phenoxyethyl, 2-Phenoxyethyl, 1-Phenoxy-propyl, 2-Phenoxy-propyl, 3-Phenoxy-propyl, 1-Phenoxy-butyl, 2-Phenoxy-butyl, 3-Phenoxy-butyl und 4-Phenoxy-butyl, besonders bevorzugt Benzoxy, 1-Phenoxyethyl und 2-Phenoxyethyl,
- C₁- bis C₂₀-Alkylamino, bevorzugt C₁- bis C₁₂-Alkylamino wie Methylamino, Ethylamino, n-Propylamino, iso-Propylamino, n-Butylamino, iso-Butylamino, sec.-Butylamino, tert.-Butylamino, n-Pentylamino, iso-Pentylamino, sec.-Pentylamino, neo-Pentylamino, 1,2-Dimethylpropylamino, n-Hexylamino, iso-Hexylamino, sec.-Hexylamino, n-Heptylamino, iso-Heptylamino, n-Octylamino, iso-Octylamino, n-Nonylamino, iso-Nonylamino, n-Decylamino, iso-Decylamino, n-Undecylamino, iso-Undecylamino, n-Dodecylamino und iso-Dodecylamino, besonders bevorzugt C₁- bis C₄-Alkylamino wie Methylamino, Ethylamino, n-Propylamino, iso-Propylamino, n-Butylamino, iso-Butylamino, sec.-Butylamino und tert.-Butylamino,
- Phenylamino,
- C₇- bis C₂₀-Phenylalkylamino, bevorzugt C₇- bis C₁₂-Phenylalkylamino wie Benzylamino, 1-Phenethylamino, 2-Phenethylamino, 1-Phenyl-propylamino, 2-Phenyl-propylamino, 3-Phenyl-propylamino, 1-Phenyl-butylamino, 2-Phenyl-butylamino, 3-Phenyl-butylamino und 4-Phenyl-butylamino, besonders bevorzugt Benzylamino, 1-Phenethylamino und 2-Phenethylamino,
- Pyridyl,
- Imidazolyl oder R¹ und R² oder R¹ und R³ oder R² und R³ gemeinsam für ein gegebenenfalls ein- bis fünffach durch C₁- bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen wie Aminoalkyl, N-Methylaminoalkyl, N,N-Dimethylaminoalkyl, N-Ethylaminoalkyl, N,N-Diethylaminoalkyl, Methoxyalkyl und Ethoxyalkyl, bevorzugt Aminoethyl, N-Methylaminoethyl, N,N-Dimethylaminoethyl, Aminopropyl, N-Methylaminopropyl, N,N-Dimethylaminopropyl, Hydroxyethyl, Methoxyethyl, Ethoxyethyl, Hydroxypropyl, Methoxypropyl und Ethoxypropyl.

Als neue 1,3-Diamine IV seien die folgenden genannt:
N,N'-2-Trimethyl-1,3-propandiamin, N,N,N',N'-Tetraethyl-2-methyl-1,3-propandiamin, N,N'-Di-n-propyl-2-methyl-1,3-propandiamin, N,N,N',N'-Tetra-n-propyl-2-methyl-1,3-propandiamin, N,N'-Di-i-propyl-2-methyl-1,3-propandiamin, N,N'-Di-n-butyl-2-methyl-1,3-propandiamin, N,N,N',N'-Tetra-n-butyl-2-methyl-1,3-propandiamin, N,N'-Dicyclohexyl-2-methyl-1,3-propandiamin, N,N'-Di-(2-ethylhexyl)-2-methyl-1,3-propandiamin, N,N,N',N'-Tetra-(2-ethylhexyl)-2-methyl-1,3-propandiamin, N,N'-Di-(2-hydroxyethyl)-2-methyl-1,3-propandiamin, N,N'-Di-(3-hydroxypropyl)-2-methyl-1,3-propandiamin, N,N'-Di-(3-methoxypropyl)-2-methyl-1,3-propandiamin, N,N'-Di-(N,N-dimethyl-2-aminoethyl)-2-methyl-1,3-propandiamin, N,N'-Di-(N,N-dimethyl-3-aminopropyl)-2-methyl-1,3-propandiamin, N,N'-Di-phenyl-2-methyl-1,3-propandiamin,N,N'-Dibenzyl-2-methyl-1,3-propandiamin, Di-(N-i-propylamino-2-methylpropyl)-1,6-hexandiamin,
N-Cyclohexyl-N'-i-propyl-2-methyl-propan-1,3-diamin, N,N-Di-n-propyl-N'-i-propyl-2-methyl-propan-1,3-diamin, N-i-Propyl-N'-phenyl-2-methyl-propan-1,3-diamin, N-i-Propyl-N'-(2-hydroxyethyl)-2-methyl-propan-1,3-diamin, N-i-Propyl-N'-(3-hydroxypropyl)-2-methyl-propan-1,3-diamin, N-i-Propyl-N'-(3-methoxypropyl)-2-methyl-propan-1,3-diamin, N-i-Propyl-N'-(3-methylaminopropyl)-2-methyl-propan-1,3-diamin, N-i-Propyl-N'-(3-dimethylaminopropyl)-2-methyl-propan-1,3-diamin, 11,14-Dimethyl-1,5,9,13-tetraaza-pentadecan, 1-Cyclohexylamino-2-methyl-3-piperidyl-propan, 2-Methyl-1-piperidyl-3-i-propylamino-propan, 2-Methyl-1-morpholino-3-i-propylamino-propan, 2-Methyl-1-morpholino-3-piperidyl-propan, 2,5,12,15-Tetramethyl-3,7,10,14-tetraaza-hexadecan, 2,5,16,19-Tetramethyl-3,7,14,18-tetraaza-eicosan, 2,5,17,20-Tetramethyl-3,7,11,15,19-pentaaza-heneicosan,
N,N'-Di-t-Butyl-1,3-propandiamin,N,N'-Di-t-Butyl-2-ethyl-1,3-propandiamin, N,N'-Di-t-Butyl-2-n-nonyl-1,3-propandiamin, N,N'-Di-i-propyl-1,3-butandiamin, N,N'-Di-n-Butyl-1,3-butandiamin, N,N'-Di-n-Butyl-2-n-propyl-1,3-pentandiamin, N,N'-Di-i-Propyl-1,3-butandiamin.

N,N'-Alkylsubstituierte 1,3-Propandiamine finden zum Beispiel Verwendung bei der Herstellung von FCKW-freien Polymeren, als Katalysatoren für Polyurethane und Härtungsmittel z.B. für Epoxidharze, als Komponenten oder Zusätze bei der Herstellung bestimmter Nylontypen, als Edukte für Polyharnstoffe oder bei der Erstellung von Appreturmitteln für Textilfasern und für Klebe- und Haftmittel.

### Beispiele

- Herstellung von ungesättigten Aminen I und anschließende Hydrierung

### Beispiel 1

26,3 g (0,375 mol) Methacrolein wurde in einer Stunde zu 88,5 g (1,5 mol) mol Isopropylamin so zugetropft, daß die Temperatur nicht über 35°C steigt. Man rührt für eine Stunde bei 40°C nach und hydriert das rohe Isopropylamin/Methacrolein Kondensationsprodukt an 3 g Palladium auf Aktivkohle (10% Pd) bei 50°C und 100 bar Wasserstoff bis zum Ende der Wasserstoffaufnahme. Die fraktionierende Destillation (Sdp.: 100°C/25 mbar) ergab 52 g (81 %) N,N'-Diisopropyl-2-methyl-1,3-propandiamin.

### Beispiel 2

26,3 g (0,375 mol) Methacrolein wurde in einer Stunde zu 88,5 g (1,5 mol) Isopropylamin so zugetropft, daß die Temperatur nicht über 45°C steigt. Man rührt für eine Stunde bei 40°C nach und hydriert das rohe Isopropylamin/Methacrolein Kondensationsprodukt an 0,5 g Palladium auf Aktivkohle (10 % Pd) bei 50°C und 100 bar Wasserstoffdruck bis zum Ende der Wasserstoffaufnahme. Die fraktionierende Destillation (Sdp.: 100°C/25 mbar) ergab 52 g (81 %) N,N'-Diisopropyl- 2-methyl-1,3-propandiamin.

### Beispiel 3

61,3 g (0,875 mol) Methacrolein wurde in einer Stunde zu 206,5 g (3,5 mol) Isopropylamin so zugetropft, daß die Temperatur nicht über 35°C steigt. Man rührt für eine Stunde bei 40°C nach und hydriert das rohe Isopropylamin/Methacrolein Kondensationsprodukt an 5 g Palladium auf Aktivkohle (10% Pd) bei 25°C und Atmosphärendruck bis zum Ende der Wasserstoffaufnahme. Die fraktionierende Destillation (Sdp.: 98°C/20 mbar) ergab 118 g (79%) N,N'-Diisopropyl-2-methyl-1,3-propandiamin.

### Beispiel 4

105 g (1,5 mol) Methacrolein wurde in einer Stunde zu 3,54 g (6 mol) Isopropylamin so zugetropft, daß die Temperatur nicht über 45°C steigt. Man rührt für eine Stunde bei 40°C nach und hydriert das rohe Isopropylamin/Methacrolein Kondensationsprodukt nach Verdünnen mit 400 ml Tetrahydrofuran an 15 g Palladium auf Aktivkohle (10 % Pd) bei 50°C und 100 bar Wasserstoff bis zum Ende der Wasserstoffaufnahme. Die fraktionierende Destillation (Sdp.: 98°C/20 mbar) ergab 212 g (83 %) N,N'-Diisopropyl-2-methyl-1,3-propandiamin.

### Beispiel 5

Analog Beispiel 1 erhielt man 310 g (83 %) N,N'-Di-n-butyl-2-methyl-1,3-propandiamin (Sdp.: 97°C/1 mbar) aus 140 g (2 mol) Methacrolein und 672 g (8 mol) n-Butylamin (Molverhältnis 1 : 4) an 15 g Palladium auf Aktivkohle (10% Pd).

### Beispiel 6

Analog Beispiel 4 erhielt man 346 g (87 %) N,N'-Di-tert.-butyl-2-methyl-1,3-propandiamin (Smp.: 58°C; Sdp.: 106°C/15 mbar) aus 140 g (2 mol) Methacrolein und 504 g (6 mol) tert.-Butylamin Molverhältnis (1 : 3) an 15 g Palladium auf Aktivkohle (10 % Pd).

### Beispiel 7

Analog Beispiel 1 erhielt man 459 g (83%) N,N'-Di-cyclohexyl-2- methyl-1,3-propandiamin (Sdp.: 150 bis 160°C/1 mbar) aus 153,5 g (2,2 mol) Methacrolein und 870 g (8,8 mol) Cyclohexylamin (Molverhältnis 1 : 4) an 15 g Palladium auf Aktivkohle (10 % Pd).

### Beispiel 8

Analog Beispiel 1 erhielt man 398 g (87 %) N,N'-Di-(2-ethylhexyl)-2-methyl-1,3-propandiamin (Sdp.: 170°C/1 mbar) aus 100 g (1,5 mol) Methacrolein und 754 g (5,8 mol) 2-Ethylhexylamin (Molverhältnis 4 : 1) an 15 g Palladium auf Aktivkohle (10 % Pd).

### Beispiel 9

Analog Beispiel 1 erhielt man 76 g (20 %) N,N'-Dibenzyl-2-methyl-1,3-propandiamin (Sdp.: 59°C/1 mbar) aus 112 g (1,6 mol) Methacrolein und 686 g (6,4 mol) Benzylamin (Molverhältnis 4 : 1) an 10 g Palladium auf Aktivkohle (10 % Pd).

### Beispiel 10

Analog Beispiel 1 erhielt man 376 g (73 %) N,N'-Di-(N,N-dimethyl-3-aminopropyl)-2-methyl-1,3-propandiamin (Sdp.: 159°C/5 mbar) aus 140 g (2 mol) Methacrolein und 816 g (8 mol) N,N-Dimethylpropylendiamin (Molverhältnis 4 : 1) an 15 g Palladium auf Aktivkohle (10 % Pd).

### Beispiel 11

Analog Beispiel 1 erhielt man 167 g (64 %) N,N'-Di-(2-aminoethyl)-2-methyl-1,3-diaminopropan (Sdp.: 118 bis 130°C/1 mbar) aus 102 g (1,5 mol) Methacrolein und 901,5 g (15 mol) Ethylendiamin (Molverhältnis 1 : 10) an 15 g Palladium auf Aktivkohle (10 % Pd).

### Beispiel 12

Analog Beispiel 1 erhielt man 310 g (59 %) N,N'-Di-(2-hydroxyethyl)-2-methyl-1,3-propandiamin (Sdp.: 162 bis 172°C/1 mbar)) aus 140 g (3 mol) Methacrolein und 488 g (8 mol) Ethanolamin (Molverhältnis 1 : 4) an 15 g Palladium auf Aktivkohle (10 % Pd).

### Beispiel 13

Analog Beispiel 1 erhielt man 350 g (69 %) N,N'-Di-(2-methoxyethyl)-2-methyl-1,3-propandiamin (Sdp.: 96°C/1 mbar) aus 175 g (2,5 mol) Methacrolein und 750 g (10 mol) Methoxyethylamin (Molverhältnis 1 : 4) an 15 g Palladium auf Aktivkohle (10 % Pd).

### Beispiel 14

Analog Beispiel 1 erhielt man 150 g (60 %) N,N'-Di-isopropyl-1,3-butandiamin (Sdp.: 43°C) aus 102 g (1,5 mol) Crotonaldehyd und 344 g (5,8 mol) Isopropylamin (Molverhältnis 1 : 4) an 10 g Palladium auf Aktivkohle (10 % Pd).

### Beispiel 15

Analog Beispiel 1 erhielt man 162 g (81 %) N,N'-Di-n-butyl-1,3-butandiamin (Sdp.: 120°C/18 mbar) aus 70 g (1 mol) Crotonaldehyd und 584 g (8 mol) n-Butylamin (Molverhältnis 1 : 8) an 15 g Palladium auf Aktivkohle (10 % Pd).

### Beispiel 16

Analog Beispiel 1 erhielt man 440 g (67 %) N,N'-Di-tert.-butyl-2-nonyl-1,3-propandiamin (Sdp.: 150°C/1 mbar) aus 546 g (2,1 mol) 2-Nonylacrolein (als 70 %ige Lösung in Decan) und 444 g (6 mol) tert.-Butylamin (Molverhältnis 1 : 3) an 15 g Palladium auf Aktivkohle (10 % Pd).

### Beispiel 17

Analog Beispiel 1 erhielt man 395 g (84 %) N,N'-Di-tert.-butyl-1,3-propandiamin (Sdp.: 132°C/100 mbar) aus 140 g (2,5 mol) Acrolein und 732 g (10 mol) tert.-Butylamin (Molverhältnis 1 : 4) an 15 g Palladium auf Aktivkohle (10 % Pd).

### Beispiel 18

Analog Beispiel 1 erhielt man 29 g (80 %) 1,3-Dipiperidyl-2-methylpropan (Sdp.: 90 bis 100°C/1 mbar) aus 10 g (0,14 mol) Methacrolein und 50 g (0,6 mol) Piperidin (Molverhältnis 1 : 4,2) an 2 g Pd auf Aktivkohle (10 % Pd).

### Beispiel 19

Analog Beispiel 1 erhielt man 250 g (40 %) N,N,N',N'-Tetra-n-butyl-2-methyl-1,3-propandiamin (Sdp.: 125°C/1 mbar) aus 140 g (2 mol) Methacrolein und 155 g (12 mol) Di-n-butylamin (Molverhältnis 1 : 6) an 15 g Pd auf Aktivkohle (10 % Pd).

### Beispiel 20

Analog Beispiel 1 erhielt man 149 g (50 %) N,N,N',N'-Tetra-n-propyl-1,3-propandiamin (Sdp.: 137°C) aus 68 g (1,2 mol) Acrolein und 740 g (7,3 mol) Di-n-propylamin (Molverhältnis 1 : 6) an 15 g Pd auf Aktivkohle (10 % Pd).

Vergleichsbeispiel:
nach J. Am. Chem. Soc. 74, 2016 bis 2018 (1952) Tabelle 1, 10. Beispiel
35 g (0,5 mol) Methacrolein wurde in einer Stunde zu einer Lösung von 25 g wasserfreiem Kaliumcarbonat in 147,5 g (2,5 mol) Isopropylamin bei einer Temperatur von 5 bis 10°C getropft. Nach Abfiltrieren des Kaliumcarbonats wurde das rohe Isopropylamin/Methacrolein Kondensationsprodukt an 5 g von Wasser befreitem Raney-Nickel bei 105°C und 70 bar Wasserstoff in 6 h hydriert. Die fraktionierende Destillation (Sdp.: 80-100°C/35 mbar) ergab 47 g (50 %) N,N'-Diisopropyl-2-methyl-1,3-diamin. Die Literaturausbeute beträgt 58 %.

Beispiele zur kontinuierlichen Hydrierung der Schiff'schen Basen:

### Beispiel 21

Ein vertikal angeordneter Rohrreaktor (Durchmesser: 14 mm, Füllhöhe: 50 cm, ölbeheizter Doppelmantel) wurde mit 75 ml (47,6 g) eines Katalysators mit 0,5 % Pd auf γ-Aluminiumoxid in Form von 4-mm-Strängen gefüllt (Katalysatorherstellung durch Porentränkung von γ-Aluminiumoxid mit wäßriger Palladiumnitrat-Lösung und Trocknen bei 120°C). Der Katalysator wird in situ reduziert.

Durch den Reaktor werden stündlich 20 g einer Lösung bestehend aus 155 g (2,6 mol) Isopropylamin und 61 g (0,88 mol) Methacrolein in 200 g Tetrahydrofuran von unten nach oben gefahren. Stündlich durchströmen 10 Nl Wasserstoff den Reak tor bei einem Druck von 80 bar. Die Temperatur beträgt 70°C Der Reaktionsaustrag wird auf Normaldruck entspannt. Die Ausbeute an N,N'-Di-isopropyl-2-methyl-1,3-diaminopropan beträgt 80 %.

### Beispiel 22

### N,N,N',N',2-Pentamethyl-1,3-diaminopropan

Ein vertikal angeordneter Rohrreaktor (Durchmesser: 14 mm, Füllhöhe: 50 cm, ölbeheizter Doppelmantel) wurde mit 75 ml (47,6 g) eines Katalysators mit 0,5 % Pd auf γ-Aluminiumoxid in Form von 4-mm-Strängen gefüllt (Katalysatorherstellung durch Porentränkung von γ-Aluminiumoxid mit wäßriger Palladiumnitrat-Lösung und Trocinen bei 120°C). Der Katalysator wird in situ reduziert.

Durch den Reaktor werden stündlich 100 ml einer 20 %igen Lösung von Methacrolein in Tetrahydrofuran und 60 ml Dimethylamin von unten nach oben gefahren. Stündlich durchströmen 10 Nl Wasserstoff den Reaktor bei einem Druck von 75 bar. Die Temperatur beträgt 70°C. Der Reaktionsaustrag wird auf Normaldruck entspannt, von Dimethylamin befreit und destilliert. Der über eine Stunde gesammelte Reaktionsaustrag ergibt nach Destillation bei 70°C/100 mbar 28 g N,N,N',N',2-Pentamethyl-1,3-diaminopropan. Die Ausbeute beträgt 85 %.
- Umaminierung der ungesättigten Aminen I und anschließende Hydrierung

### Beispiel 23

168 g (2,4 mol) wurden in einer Stunde zu 354 g (6 mol) Isopropylamin so zugetropft, daß die Temperatur nicht über 35°C steigt. Man rührt für eine Stunde bei 40°C nach. Dieses rohe Isopropylamin/Methacrolein-Kondensationsprodukt wird bei 40°C mit 287 g (2,9 mol) Cyclohexylamin versetzt und gleichzeitig Isopropylamin abdestilliert. Das so erhaltene, unsymmetrisch substituierte Diaminvorprodukt wird mit 400 ml Tetrahydrofuran verdünnt und an 15 g Pd auf Aktivkohle (10 % Pd) bei 50°C und 100 bar bis zum Ende der Wasserstoffaufnahme hydriert. Die fraktionierte Destillation (Sdp.: 99 bis 106°C/1 mbar) ergab 311 g (73 %) N-Isopropyl-N'-cyclohexyl-2-methyl-1,3-propandiamin.

### Beispiel 24

Analog Beispiel 23 erhielt man 191 g (52 %) 11,14-Dimethyl-1,5,9,13-tetraazapentadecan (Sdp.: 188 bis 190°C/5 mbar) aus 105 g (1,5 mol) Methacrolein, 267 g (4,5 mol) Isopropylamin und 196,5 g (1,5 mol) Dipropylentriamin (Molverhältnis 1 : 3 : 3) an 15 g Pd auf Aktivkohle (10 % Pd) und in 400 ml Tetrahydrofuran.

### Beispiel 25

Analog Beispiel 23 erhielt man 63 g (52 %) Di-(N-isopropylamino-2-methyl-propyl)-1,6-hexandiamin (Sdp.: 170°C/1 mbar) aus 50 g (0,71 mol) Methacrolein, 176 g (2,98 mol) Isopropylamin und 41,5 g (0,35 mol) Hexamethylendiamin (Molverhältnis 2 : 8 : 1) an 5 g Palladium auf Aktivkohle (10 % Pd) und in 50 ml Tetrahydrofuran.

### Beispiel 26

Analog Beispiel 23 erhielt man 171 g (51 %) N-Isopropyl-N'-(N-methyl-3-aminopropyl)-2-methyl-1,3-propandiamin (Sdp.: 90 bis 96°C/1 mbar) aus 116 g (1,7 mol) Methacrolein, 245 g (4,2 mol) Isopropylamin und 176 g (2 mol) N-Methyl-propylendiamin (Molverhältnis 1 : 2,5 : 1,2) an 10 g Palladium auf Aktivkohle (10 % Pd) und in 400 ml Tetrahydrofuran.

### Beispiel 27

Analog Beispiel 23 erhielt man 61 g (47 %) 1-Isopropylamino-2-methyl-3-morpholino-propan (Sdp.: 75°C) aus 61,3 g (0,78 mol) Methacrolein, 206 g (3,5 mol) Isopropylamin und 76 g (0,88 mol) Morpholin (Molverhältnis 1 : 4 : 1) an 10 g Palladium auf Aktivkohle (10 % Pd).

### Beispiel 28

Analog Beispiel 23 erhielt man 56 g (50 %) 1-Cyclohexylamino-2-methyl-3-piperidyl-propan (Sdp.: 120°C/1 mbar) aus 35 g (0,5 mol) Methacrolein, 170 g (2 mol) Piperidin und 43,5 g (0,5 mol) Cyclohexylamin (Molverhältnis 1 : 4 : 1) an 3 g Pd auf Aktivkohle (10 % Pd) und in 50 ml Tetrahydrofuran.

### Beispiel 29

Analog Beispiel 23 erhielt man 17 g (16%) 1-Morpholino-2-methyl-3-piperidylpropan (Sdp.: 110°C/1 mbar) aus 35 g (0,5 mol) Methacrolein, 170 g (2 mol) Piperidin und 43,5 g (0,5 mol) Morpholin (Molverhältnis 1 : 4 : 1) an 3 g Pd auf Aktivkohle (10 % Pd).

## Patentansprüche

1. Verfahren zur Herstellung von ungesättigten Aminen der allgemeinen Formel I 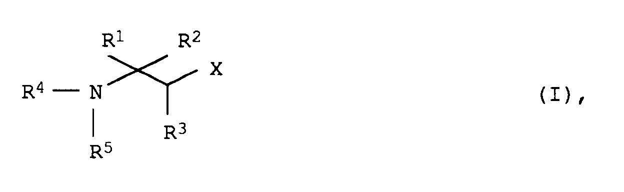 in der
R¹,R²,R³,R⁴,R⁵ Wasserstoff, C₁- bis C₂₀-Alkyl, C₂- bis C₂₀-Alkenyl, C₃- bis C₈-Cycloalkyl, C₃-bis C₈-Cycloalkenyl, Phenyl, C₇- bis C₂₀-Phenylalkyl, C₁- bis C₂₀- Alkoxy, Phenoxy, C₇- bis C₂₀-Phenoxyalkyl, C₁-bis C₂₀-Alkylamino, Phenylamino, C₇- bis C₂₀-Phenylalkylamino, Pyridyl, Imidazolyl oder R¹ und R² oder R¹ und R³ oder R² und R³ gemeinsam für ein gegebenenfalls einbis fünffach durch C₁- bis C₄-Alkyl oder gegebenenfalls ein- bis vierfach durch Amino, N-C₁- bis C₈-Alkylamino, N,N-C₁-bis C₈-Dialkylamino, Hydroxy, O-C₁- bis C₈-Alkylamino und/oder C₁- bis C₈-Alkoxy-carbonyl substituiertes C₂- bis C₈-Alkylen und
X =C-N(R⁴R⁵) oder -C=N-R⁴,
durch Umsetzung von 2-Alkenalen der allgemeinen Formel II 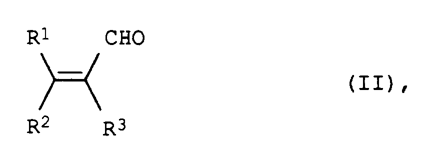 in der R¹, R² und R³ die oben genannten Bedeutungen haben, mit Ammoniak, primären oder sekundären Aminen der allgemeinen Formel III 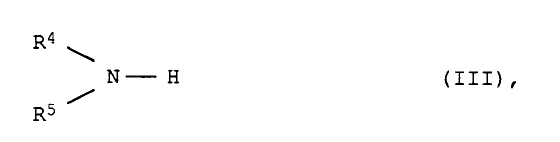 in der R⁴ und R⁵ die oben genannten Bedeutungen haben, bei Temperaturen von (-20) bis 70°C und Drücken von 0,01 bis 100 bar, dadurch gekennzeichnet, daß man die Umsetzung ohne Abtrennung des Reaktionswassers durchführt.

2. Verfahren zur Umsetzung von ungesättigten Aminen I gemäß Anspruch 1 zu den ungesättigten Aminen I' 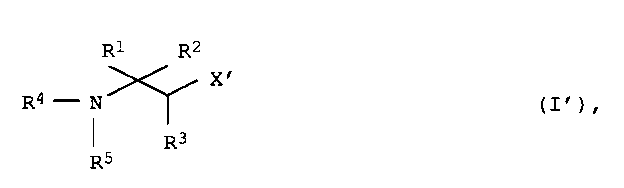 in der R¹, R², R³, R⁴ und R⁵ die oben genannten Bedeutungen haben und X' für =C-N(R⁶R⁷) oder -C=N-R⁶ steht und R⁶ und R⁷ die für R¹ bis R⁵ angegebenen Bedeutungen hat, dadurch gekennzeichnet, daß man die ungesättigten Amine I mit primären oder sekundären Aminen der allgemeinen Formel III' 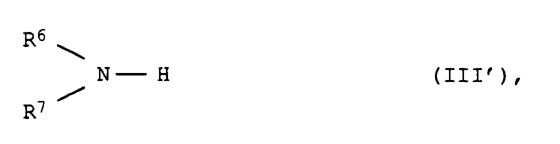 in der R⁶ und R⁷ die für R¹ bis R⁵ angegebenen Bedeutungen hat, bei Temperaturen von 20 bis 150°C umsetzt.

3. Verfahren zur Hydrierung von ungesättigten Aminen I oder I' gemäß Anspruch 1 oder 2, dadurch gekennzeichnet, daß man die ungesättigten Amine I oder I' unter einem Wasserstoffdruck von 1 bis 350 bar bei Temperaturen von 0 bis 150°C hydriert.

4. 1,3-Diamine der allgemeinen Formel IV 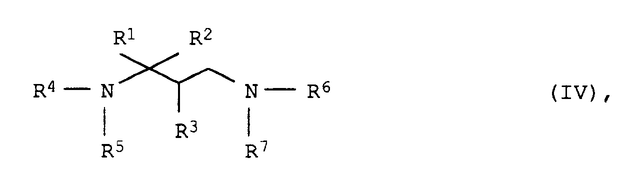 in der
R¹,R²,R³,R⁴,R⁵,R⁶,R⁷
Wasserstoff, C₁- bis C₂₀-Alkyl, C₂- bis C₂₀-Alkenyl, C₃-bis C₈-Cycloalkyl, C₃- bis C₈-Cycloalkenyl, Phenyl, C₇-bis C₂₀-Phenylalkyl, C₁- bis C₂₀- Alkoxy, Phenoxy, C₇-bis C₂₀-Phenoxyalkyl, C₁- bis C₂₀-Alkylamino, Phenylamino, C₇- bis C₂₀-Phenylalkylamino, Pyridyl, Imidazolyl oder R¹ und R² oder R¹ und R³ oder R² und R³ gemeinsam für ein gegebenenfalls ein- bis fünffach durch C₁- bis C₄-Alkyl oder gegebenenfalls ein- bis vierfach durch Amino, N-C₁- bis C₈-Alkylamino, N,N-C₁- bis C₈-Dialkylamino, Hydroxy, O-C₁- bis C₈-Alkylamino und/oder C₁- bis C₈-Alkoxycarbonyl substituiertes C₂- bis C₈-Alkylen
bedeuten, mit der Maßgabe, daß R¹ und R² nicht für Wasserstoff stehen, wenn R³ Methyl und R⁴ und R⁵, R⁶ und R⁷ jeweils Wasserstoff oder Methyl, R⁴ und R⁶ Wasserstoff und R⁵ und R⁷ Ethyl sowie R⁴ und R⁵, R⁶ und R⁷ jeweils zusammen (CH₂)₅ oder (CH₂)₂-O-(CH₂)₂ bedeuten.
